# EUROPEAN PATENT SPECIFICATION

(11) **EP 1 161 672 B1**
(45) Date of publication and mention of the grant of the patent: **25.10.2006**
(21) Application number: 00909531.6
(22) Date of filing: 15.03.2000
(51) Int. Cl.: G01N 17/00

(54) **A DEVICE FOR DETECTING THE PRESENCE OF CHEMICAL CONTAMINANTS**
EINE ANORDNUNG ZUR DETEKTION DER ANWESENHEIT VON CHEMISCHEN VERUNREINIGUNGEN
DISPOSITIF PERMETTANT DE DETECTER LA PRESENCE DES CONTAMINANTS CHIMIQUES

(30) Priority: 16.03.1999 GB 9906014
(43) Date of publication of application: 12.12.2001
(73) Proprietor: Masstech International Limited, Crowthorne, Berkshire RG45 6QR (GB)
(72) Inventor: BRIDGES, Robert, Crowthorne, Berkshire RG45 6QR (GB)
(74) Representative: Draper, Martyn John
(86) International application number: PCT/GB2000/000959
(87) International publication number: WO 2000/055598

(56) References cited:
- WO-A-88/01052
- FR-A- 2 704 318
- US-A- 3 846 795
- US-A- 4 237 972
- US-A- 4 271 120
- US-A- 4 335 615
- US-A- 4 628 252
- US-A- 5 728 943
- PATENT ABSTRACTS OF JAPAN vol. 006, no. 148 (P-133), 7 August 1982 (1982-08-07) & JP 57 067843 A (TOKYO KOKI SEIZOSHO:KK), 24 April 1982 (1982-04-24)

## Description

The present invention relates to a device such as an indicator, switch or actuator for detecting the presence of a chemical contaminant. Such a detector is used, for example, in filling stations and chemical plants to detect the presence of unwanted chemicals.

Chemical detection devices are frequently electronic and therefore have to be designed to be intrinsically safe making them prohibitively expensive for many applications and are dependent on the reliability of the power supply. Further, recent surveys in filling stations have shown that many of these devices have been physically disabled or no longer function for other reasons.

US3846795 discloses apparatus for providing an early warning of impending failure of a system structural element subject to corrosion. The apparatus provides a housing containing a corrodible member and coupled to the structure so as to expose the corrodible member to the same corrosive environment as in the system. When the specimen cracks and fails, a spring drives an upper support upward, carrying with it a magnetic member which is sensed. The corrosive activity is due to impurities in e.g. pure cooling water such as high salt content, reactive halogens and halides, MgCl2, NaCl and the system is for use in the chemical industry where corrosive fluids are present. The shortened lifetime of the corrodible member can be attributed to the presence of a contaminant e.g. chlorine.

The present invention aims to provide a low cost, disposable device which does not require electricity.

According to a first aspect of the present invention there is provided a device for detecting the presence of a chemical leakage, the device comprising an indicator element which is held in a first position by means of a failure element which is held in tension, the failure element being made of a material which fails in the presence of the chemical leakage, thereby releasing the indicator element from its first position and allowing it to move into a second position in order to provide an indication of the presence of the leakage, wherein the failure element comprises a number of different materials arranged in series and/or in parallel.

According to a second aspect of the present invention, which may be independent of, or used in conjunction with the first aspect of the invention, there is provided A device for detecting the presence of a chemical leakage, the device comprising a resilient indicator element which is held in a first position and is anchored in the first position by means of a failure element, the failure element being made of a material which fails in the presence of the chemical leakage, whereby releasing the indicator element from its first position and allowing it to move into a second position in order to provide an indication of the presence of the leakage; wherein the failure element is elongate in the sense that it is larger in the direction in which the indicator element moves on failure of the failure element than it is in any other dimension, and wherein the failure element comprises a number of different materials arranged in series and/or in parallel.

The device is based on simple chemistry and therefore cannot fail to work upon contact with the chemical to be detected. The device can be made very simple in construction allowing it to be produced very cheaply.

With different materials arranged in series, the device will operate when any one of a number of contaminants to which a single material is responsive, is present. A parallel arrangement, on the other hand, will only fail when contaminants to which all of the materials are responsive are present. With a combination of series and parallel materials, a device can be tailored to detect a sophisticated selection of contaminants.

At present, when monitoring wells, it is necessary to take a sample from every well to determine whether or not contamination has occurred. With the present invention it would only be necessary to take samples from wells where the element has failed.

With the first aspect of the invention, as the failure element is held in tension, a failure, no matter how small, anywhere along the failure element, will cause the device to indicate the presence of a contaminant.

With the second aspect of the invention, as the failure element is elongate (for example, the length of the failure element in the direction in which the indicator element moves on failure is at least 3 times, preferably at least 10 times, more preferably at least 20 times, and most preferably at least 50 times its size in any other dimension), it can be extended across a zone in which chemical contaminant is to be detected, thereby providing a cost effective way of detection beyond a single location.

The indicator element may be held in the first position by a biasing force, the biasing force acting to move the indicator element to the second position upon failure of the failure element. Alternatively, the indicator element is held in the first position by a biasing force and wherein a further force, which is strong enough to override the biasing force is arranged to act on the indicator element to move it to the second position upon failure of the failure element.

In its simplest form the indicator element is a spring which is fixed to the failure element, the spring being under compression, such that the failure element is under tension. The failure element is preferably a tubular member. In order to provide a further degree of monitoring of the condition of the failure element, the tubular member is preferably sealed, the inside of the tubular member is maintained at a pressure other than atmospheric, and means are provided to monitor this pressure to determine the integrity of the tubular member. In order to operate a valve, a cable can pass through the tubular member and be fixed to one end of the spring such that, on failure of the failure element the cable is pulled through the tubular member to operate the valve. The spring is preferably attached to the failure element by a respective washer at each end of the spring, each washer being anchored to the failure element so as to be capable of movement in only one direction along the failure element. This allows the indicator element to be fastened in place with the necessary preload by pushing each washer along the failure element. The washer will then be held in place as the spring will tend to urge it in the direction in which it cannot move.

In order to assist with an assessment of the full extent of a chemical contaminant, the failure element is preferably made of a material which changes its appearance in the present of the contaminant.

Preferably, the failure element is a tubular element and the indicator element is within the tubular element and is fixed at one end to the failure element, while its other end projects beyond the other end of the failure element and is biased away from the other end of the failure element.

The further force may be any type of force, such as a magnetic force. However, preferably, the failure element and indicator element are arranged to be supported vertically, wherein the further force is gravity.

In an alternative arrangement the indicator element comprises a core surrounded by a sleeve, the sleeve being biased away from the core, wherein the failure element holds the sleeve in a position in which it surrounds the core, whereby, when the failure element fails, the sleeve is released and moved away from the core providing a visual indication of the presence of the contaminant. Preferably, the outer surface of the sleeve is a different colour from the outer surface of the core, thereby improving the visual indication of the presence of a contaminant.

In addition to the provision of visual indication, the device can also be arranged to operate some failsafe mechanism to prevent further contamination, such as by automatically closing a valve.

In order to detect the presence of a contaminant several of the devices described above are arranged over the area, preferably in parallel. This allow a map of the location and extent of a contaminant to be created.

Examples of devices will now be described with reference to the accompanying drawings, in which:
Fig. 1 is a schematic cross-section of a first example;
Fig. 2 is a cross-section of a second example;
Fig. 3 is a cross-section of a ground water monitoring well having a device;
Fig. 4 shows a device positioned under a tank in order to monitor leakage from the tank;
Fig. 4A is a detail of the part ringed as 4A in Fig. 4; and
Fig. 5 is a schematic plan view showing the operation of Fig. 4 to determine the full extent of contaminant leakage.

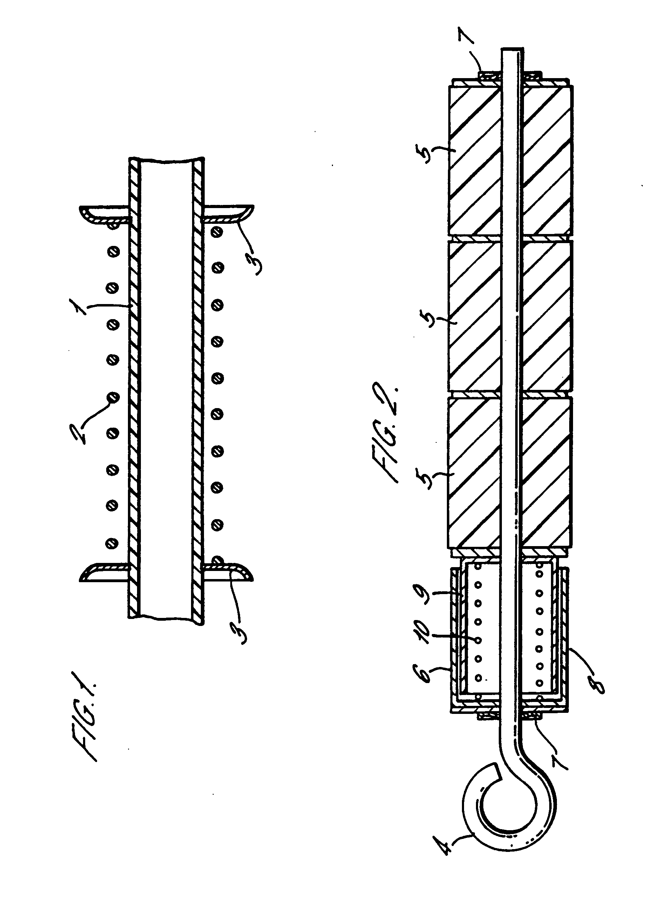

The device shown in Fig 1 comprises a tubular member 1 of high-density polystyrene. Attached to the tubular member 1 is a spring 2 which is held in compression by a pair of starlock washers 3 which anchor it to the tubular member 1. In the presence of a chemical contaminant, in this case any petroleum product, the tubular member 1 will fail and the spring 2 extends. The extension of the spring can be used to trigger a mechanical signal or alarm, or can close valves. A cable, such as a bowden cable, may extend through the tubular element and be fixed to one end of the starlock washers. On failure of the tubular member, the cable will be pulled through the tubular element allowing operation of a valve. The failure made may be shearing, stretching or bending of the tubular member, but is more likely to be slippage of a starlock washer when the surface of the tubular member has been degraded by the contaminant.

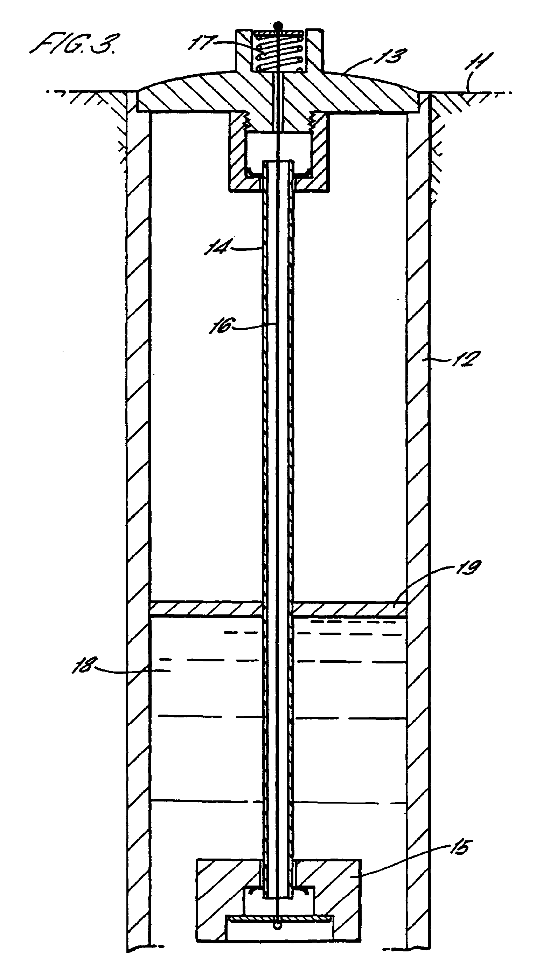

A second example is shown in Fig 2. In this case, the device is built around a central rod 4 on which are threaded three failure elements 5 of expanded polystyrene, and a resilient indicator element 6. The failure elements 5 and resilient indicator element 6 are held in place by a pair of starlock washers 7. The resilient indicator element 6 comprises a sleeve 8 surrounding a core 9. The sleeve 8 and core 9 are biased away from one another by compression spring 10. When any one of the failure elements 5 fails in the presence of a chemical contaminant, the compression of spring 10 forces the sleeve 8 and core 9 axially away from one another, so that the outer surface of the core 9 is uncovered providing a visual indication that failure has occurred.

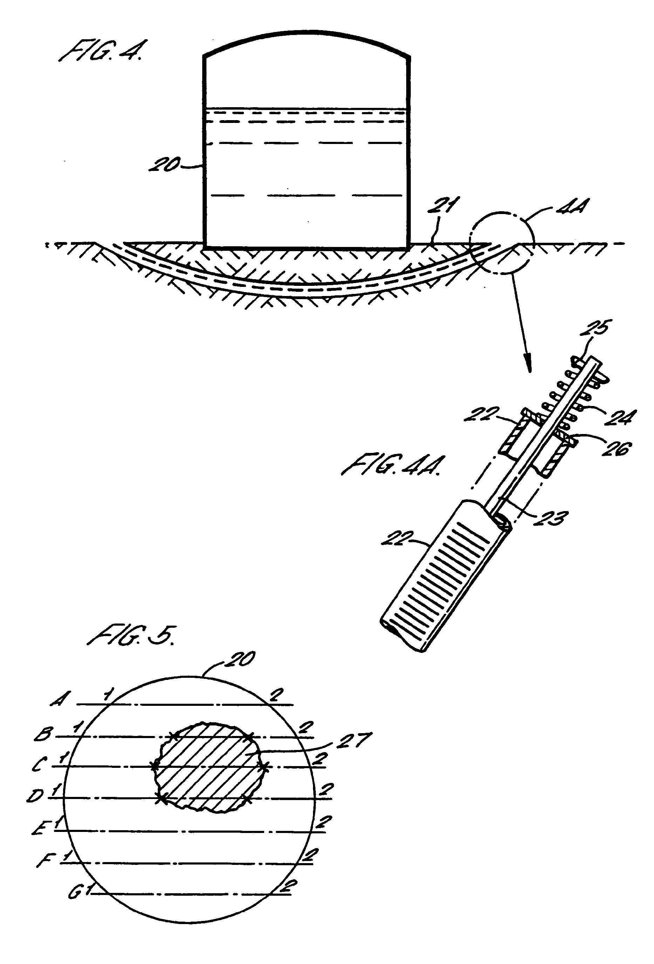

A third example is shown in Fig. 3. This shows a device used in a ground water monitoring well which is buried below ground level 11. The well comprises a slotted or perforated well casing 12 closed at its top end by a well monitoring cap 13. In this case, the failure element 14 is a tubular member which is suspended from the cap 13 and fixed at its bottom end to a weight 15. The indicator element comprises an elongate rod 16 extending within the failure element 14 and attached at its lower end to the weight 15, and a spring 17 mounted in the cap 13 and acting to bias the rod 16 upwardly.

The lower part of the well is filled with ground water 18. If a chemical contaminant 19 is present floating on the ground water 18, this will react chemically with the failure member 14 causing it to fail. At this time, the full mass of the weight 15 is held by the rod 16 which will be pulled downwardly under gravity compressing spring 17 and providing a visual indication at the well cap of the presence of the contaminant.

When monitoring a well, the device could be several metres long, so that no matter at what level the hydrocarbon contaminant existed, the element would fail.

A fourth example is shown in Figs. 4, 4A and 5. This example is designed to detect leakage from a tank 20. A plurality of elongate devices are buried in the ground 21 beneath the tank 20. Fig. 4 shows one such indicator, and the arrangement of all of the indicators is shown in plan in Fig. 5. The device comprises a perforated tube 22 containing elongate failure element 23 which projects from one end of the perforated tube 22 and is fastened at the other end of the tube 22. A spring 24 providing the indicator element is provided to bias a starlock washer 25 attached to the end of the failure element 23 away from a washer 26 at the end of the perforated tube 22 hence holding the failure element in tension.

When a chemical contaminant 27 leaks from the tank 20, it will enter the perforated tubes 22 immediately below the leak and will cause certain failure elements 23 to fail. Once a failure element 23 fails at any location, the spring 24 which is held in compression, will push the starlock washer 25 away from the end of the tube 22 thereby providing a visual indication of the presence of a contaminant. Once a contaminant is detected, all of the failure elements 23 can be pulled out and inspected. If they are made of a material which changes its appearance or is entirely obliterated in the presence of the contaminant, it is possible to build up a map showing the location and extent of the contaminant 27 as shown in Fig. 5.

## Claims

1. A device for detecting the presence of a chemical leakage, the device comprising an indicator element which is held in a first position by means of a failure element which is held in tension, the failure element being made of a material which fails in the presence of the chemical leakage, thereby releasing the indicator element from its first position and allowing it to move into a second position in order to provide an indication of the presence of the leakage, wherein the failure element comprises a number of different materials arranged in series and/or in parallel.

2. A device according to claim 1, wherein the indicator element is held in the first position by a biasing force, the biasing force acting to move the indicator element to the second position upon failure of the failure element.

3. A device according to claim 2, wherein the biasing force is provided by the resilience of the indicator element.

4. A device according to claim 3, wherein the resilient indicator element is a spring which is fixed to the failure element, the spring being under compression, such that the failure element is under tension.

5. A device according to any preceding claim, wherein the failure element is a tubular member.

6. A device according to claim 5, wherein the tubular member is sealed, the inside of the tubular member is maintained at a pressure other than atmospheric, and means are provided to monitor this pressure to determine the integrity of the tubular member.

7. A device according to claim 4, wherein the spring is attached to the failure element by a respective starlock washer at each end of the spring each washer being anchored to the failure element so as to be capable of movement in only one direction along the failure element.

8. A device according to claim 1, wherein the failure element is made of material which changes its appearance in the presence of the leakage.

9. A device according to claim 1, wherein the indicator element is held in the first position by a biasing force and wherein a further force, which is strong enough to override the biasing force is arranged to act on the indicator element to move it to the second position upon failure of the failure element.

10. A device according to claim 9, wherein the failure element is a tubular element and the indicator element is within the tubular element and is fixed at one end to the failure element, while its other end projects beyond the other end of the failure element and is biased away from the other end of the failure element.

11. A device according to claim 9, wherein the failure element and indicator element are arranged to be supported vertically, wherein the further force is gravity.

12. A device for detecting the presence of a chemical leakage, the device comprising a resilient indicator element which is held in a first position and is anchored in the first position by means of a failure element, the failure element being made of a material which fails in the presence of the chemical leakage, whereby releasing the indicator element from its first position and allowing it to move into a second position in order to provide an indication of the presence of the leakage; wherein the failure element is elongate in the sense that it is larger in the direction in which the indicator element moves on failure of the failure element than it is in any other dimension, and wherein the failure element comprises a number of different materials arranged in series and/or in parallel.

13. A device according to claim 12, wherein the failure element is held in tension.

14. A device according to claim 12, wherein the indicator element is held in the first position by a biasing force, the biasing force acting to move the indicator element to the second position upon failure of the failure element.

15. A device according to claim 14, wherein the biasing force is provided by the resilience of the indicator element.

16. A device according to claim 15, wherein the resilient indicator element is a spring which is fixed to the failure element, the spring being under compression, such that the failure element is under tension.

17. A device according to claim 12, wherein the failure element is a tubular member.

18. A device according to claim 17, wherein the tubular member is sealed, the inside of the tubular member is maintained at a pressure other than atmospheric, and means are provided to monitor this pressure to determine the integrity of the tubular member.

19. A device according to claim 16, wherein the spring is attached to the failure element by a respective starlock washer at each end of the spring each washer being anchored to the failure element so as to be capable of movement in only one direction along the failure element.

20. A device according to claim 12, wherein the failure element is made of a material which changes its appearance in the presence of the contaminant.

21. A device according to claim 12, wherein the indicator element is held in the first position by a biasing force and wherein a further force, which is strong enough to override the biasing force is arranged to act on the indicator element to move it to the second position upon failure of the failure element.

22. A device according to claim 21, wherein the failure element is a tubular element and the indicator element is within the tubular element and is fixed at one end to the failure element, while its other end projects beyond the other end of the failure element and is biased away from the other end of the failure element.

23. A device according to claim 22, wherein the failure element and indicator element are arranged to be supported vertically, wherein the further force is gravity.

24. An arrangement for detecting the presence of a chemical leakage over a predetermined area, the arrangement comprising a plurality of devices according to any one of the preceding claims arranged over the area.

25. An arrangement according to claim 24, wherein the devices are arranged substantially in parallel.

26. A method of detecting a chemical leakage comprising the steps of positioning a device according to claim 1 in a site of potential chemical leakage and monitoring the failure element to determine when it has moved to the second position indicating the presence of a leak.

27. A method of detecting a chemical leakage comprising the steps of positioning an arrangement according to claim 24 in a site of potential chemical leakage and monitoring the failure element to determine when it has moved to the second position indicating the presence of a leak.

28. A method of detecting leaks from a vessel in a filling station containing a potential source of chemical contaminant, the method comprising the steps of positioning a device according to claim 1 in the ground beneath the vessel; and monitoring the failure element to determine when it has moved to a second position indicating the presence of a leak.

29. A method of detecting leaks from a vessel in a filling station containing a potential source of chemical contaminant, the method comprising the steps of positioning an arrangement according to claim 24 in the ground beneath a vessel; and monitoring each failure element to determine when it has moved to a second position indicating the presence of a leak.

## Patentansprüche

1. Vorrichtung zur Erfassung des Vorhandenseins einer Chemikalienleckage, wobei die Vorrichtung ein Anzeigeeinrichtungselement umfasst, welches mittels eines unter Spannung gehaltenen Fehlerelements in einer ersten Position gehalten ist, wobei das Fehlerelement aus einem Material gebildet ist, welches beim Vorhandensein der Chemikalienleckage versagt, wodurch das Anzeigeeinrichtungselement von seiner ersten Position aus freigegeben wird und ihm gestattet wird, sich zu einer zweiten Position zu bewegen, um eine Anzeige des Vorhandenseins der Leckage bereitzustellen, wobei das Fehlerelement eine Anzahl von unterschiedlichen Materialien umfasst, welche in Reihe und/oder parallel angeordnet sind.

2. Vorrichtung nach Anspruch 1, wobei das Anzeigeeinrichtungselement in der ersten Position durch eine Vorspannkraft gehalten ist, wobei die Vorspannkraft derart wirkt, dass sie das Anzeigeeinrichtungselement bei einem Versagen des Fehlerelements zu der zweiten Position bewegt.

3. Vorrichtung nach Anspruch 2, wobei die Vorspannkraft durch die Elastizität des Anzeigeeinrichtungselements bereitgestellt ist.

4. Vorrichtung nach Anspruch 3, wobei das elastische Anzeigeeinrichtungselement eine Feder ist, welche an dem Fehlerelement festgelegt ist, wobei die Feder unter Druck steht, sodass das Fehlerelement unter Spannung ist.

5. Vorrichtung nach einem der vorhergehenden Ansprüche, wobei das Fehlerelement ein rohrartiges Element ist.

6. Vorrichtung nach Anspruch 5, wobei das rohrartige Element abgedichtet ist, der Innenbereich des rohrartigen Elements bei einem von der Atmosphäre verschiedenen Druck gehalten ist und Mittel vorgesehen sind, um diesen Druck zu überwachen, um die Unversehrtheit des rohrartigen Elements zu bestimmen.

7. Vorrichtung nach Anspruch 4, wobei die Feder an dem Fehlerelement durch eine jeweilige Sternverriegelungsscheibe an jedem Ende der Feder angebracht ist, wobei jede Scheibe an dem Fehlerelement derart verankert ist, dass es zu einer Bewegung in lediglich einer Richtung entlang des Fehlerelements in der Lage ist.

8. Vorrichtung nach Anspruch 1, wobei das Fehlerelement aus einem Material hergestellt ist, welches seine äußere Erscheinung beim Vorhandensein der Leckage ändert.

9. Vorrichtung nach Anspruch 1, wobei das Anzeigeeinrichtungselement in der ersten Position durch eine Vorspannkraft gehalten ist und wobei eine weitere Kraft, welche stark genug ist, die Vorspannkraft wirkungslos zu machen, derart angeordnet ist, dass sie auf das Anzeigeeinrichtungselement wirkt, um es beim Versagen des Fehlerelements zu der zweiten Position zu bewegen.

10. Vorrichtung nach Anspruch 9, wobei das Fehlerelement ein rohrförmiges Element ist und das Anzeigeeinrichtungselement sich in dem rohrartigen Element befindet und an einem Ende an dem Fehlerelement festgelegt ist, während sein anderes Ende über das andere Ende des Fehlerelements hinausragt und von dem anderen Ende des Fehlerelements weg vorgespannt ist.

11. Vorrichtung nach Anspruch 9, wobei das Fehlerelement und das Anzeigeeinrichtungselement derart angeordnet sind, dass sie in vertikaler Richtung abgestützt sind, wobei die weitere Kraft die Schwerkraft ist.

12. Vorrichtung zur Erfassung des Vorhandenseins einer Chemikalienleckage, wobei die Vorrichtung ein elastisches Anzeigeeinrichtungselement umfasst, welches in einer ersten Position gehalten ist und in der ersten Position mittels eines Fehlerelements verankert ist, wobei das Fehlerelement aus einem Material hergestellt ist, welches beim Vorhandensein der Chemikalienleckage versagt, wodurch das Anzeigeeinrichtungselement aus seiner ersten Position gelöst wird und ihm gestattet wird, sich in eine zweite Position zu bewegen, um eine Anzeige des Vorhandenseins der Leckage bereitzustellen; wobei das Fehlerelement in dem Sinne länglich ist, dass es in der Richtung, in welcher sich das Anzeigeeinrichtungselement bei einem Versagen des Fehlerelements bewegt, größer ist als in einer beliebigen anderen Abmessung, und wobei das Fehlerelement eine Anzahl von unterschiedlichen Materialien umfasst, welche in Reihe und/oder parallel angeordnet sind.

13. Vorrichtung nach Anspruch 12, wobei das Fehlerelement unter Spannung gehalten ist.

14. Vorrichtung nach Anspruch 12, wobei das Anzeigeeinrichtungselement in der ersten Position durch eine Vorspannkraft gehalten ist, wobei die Vorspannkraft derart wirkt, dass sie das Anzeigeeinrichtungselement bei einem Versagen des Fehlerelements zu der zweiten Position bewegt.

15. Vorrichtung nach Anspruch 14, wobei die Vorspannkraft durch die Elastizität des Anzeigeeinrichtungselements bereitgestellt ist.

16. Vorrichtung nach Anspruch 15, wobei das elastische Anzeigeeinrichtungselement eine Feder ist, welche an dem Fehlerelement festgelegt ist, wobei die Feder unter Druck steht, sodass das Fehlerelement unter Spannung ist.

17. Vorrichtung nach Anspruch 12, wobei das Fehlerelement ein rohrartiges Element ist.

18. Vorrichtung nach Anspruch 17, wobei das rohrartige Element abgedichtet ist, der Innenbereich des rohrartigen Elements bei einem von der Atmosphäre verschiedenen Druck gehalten ist und Mittel vorgesehen sind, um diesen Druck zu überwachen, um die Unversehrtheit des rohrartigen Elements zu bestimmen.

19. Vorrichtung nach Anspruch 16, wobei die Feder an dem Fehlerelement durch eine jeweilige Sternverriegelungsscheibe an jedem Ende der Feder angebracht ist, wobei jede Scheibe an dem Fehlerelement derart verankert ist, dass es zu einer Bewegung in lediglich einer Richtung entlang des Fehlerelements in der Lage ist.

20. Vorrichtung nach Anspruch 12, wobei das Fehlerelement aus einem Material hergestellt ist, welches seine äußere Erscheinung beim Vorhandensein der Verschmutzung ändert.

21. Vorrichtung nach Anspruch 12, wobei das Anzeigeeinrichtungselement in der ersten Position durch eine Vorspannkraft gehalten ist und wobei eine weitere Kraft, welche stark genug ist, die Vorspannkraft wirkungslos zu machen, derart angeordnet ist, dass sie auf das Anzeigeeinrichtungselement wirkt, um es beim Versagen des Fehlerelements zu der zweiten Position zu bewegen.

22. Vorrichtung nach Anspruch 21, wobei das Fehlerelement ein rohrförmiges Element ist und das Anzeigeeinrichtungselement sich in dem rohrartigen Element befindet und an einem Ende an dem Fehlerelement festgelegt ist, während sein anderes Ende über das andere Ende des Fehlerelements hinausragt und von dem anderen Ende des Fehlerelements weg vorgespannt ist.

23. Vorrichtung nach Anspruch 22, wobei das Fehlerelement und das Anzeigeeinrichtungselement derart angeordnet sind, dass sie in vertikaler Richtung abgestützt sind, wobei die weitere Kraft die Schwerkraft ist.

24. Anordnung zur Erfassung des Vorhandenseins einer Chemikalienleckage über einen vorbestimmten Bereich, wobei die Anordnung eine Mehrzahl von Vorrichtungen nach einem der vorhergehenden Ansprüche umfasst, die über den Bereich hinweg angeordnet sind.

25. Anordnung nach Anspruch 24, wobei die Vorrichtungen im Wesentlichen parallel angeordnet sind.

26. Verfahren einer Erfassung einer Chemikalienleckage, umfassend die Schritte einer Positionierung einer Vorrichtung nach Anspruch 1 an einer Stelle einer potenziellen Chemikalienleckage sowie Überwachen des Fehlerelements, um zu bestimmen, wenn es sich unter Anzeige des Vorhandenseins eines Lecks zu der zweiten Position bewegt hat.

27. Verfahren einer Erfassung einer Chemikalienleckage, umfassend die Schritte eines Positionierens einer Anordnung nach Maßgabe von Anspruch 24 an einer Stelle einer potenziellen Chemikalienleckage sowie Überwachen des Fehlerelements, um zu bestimmen, wenn es sich unter Anzeige des Vorhandenseins eines Lecks zu der zweiten Position bewegt hat.

28. Verfahren einer Erfassung von Lecks aus einem Gefäß in einer Tankstelle, welches eine potenzielle Quelle einer chemischen Verschmutzung enthält, wobei das Verfahren die Schritte eines Positionierens einer Vorrichtung nach Anspruch 1 im Boden unter dem Gefäß; sowie ein Überwachen des Fehlerelements umfasst, um zu bestimmen, wenn es sich unter Anzeige des Vorhandenseins eines Lecks zu einer zweiten Position bewegt hat.

29. Verfahren einer Erfassung von Lecks aus einem Gefäß in einer Tankstelle, welches eine potenzielle Quelle chemischer Verschmutzung enthält, wobei das Verfahren die Schritte eines Positionierens einer Anordnung nach Maßgabe von Anspruch 24 im Boden unter einem Gefäß; sowie ein Überwachen eines jeden Fehlerelements umfasst, um zu bestimmen, wenn es sich unter Anzeige des Vorhandenseins eines Lecks zu einer zweiten Position bewegt hat.

## Revendications

1. Dispositif destiné à détecter la présence d'une fuite chimique, le dispositif comprenant un élément indicateur qui est maintenu dans une première position au moyen d'un élément de rupture qui est maintenu sous tension, l'élément de rupture étant constitué d'un matériau qui se casse en présence de la fuite chimique, libérant ainsi l'élément indicateur de sa première position et lui permettant ainsi de se déplacer dans une seconde position afin de fournir une indication de la présence de la fuite, dans lequel l'élément de rupture comprend un nombre de différents matériaux agencés en série et/ou en parallèle.

2. Dispositif selon la revendication 1, dans lequel l'élément indicateur est maintenu dans la première position par une force de déviation, la force de déviation agissant pour déplacer l'élément indicateur dans la seconde position lors de la rupture de l'élément de rupture.

3. Dispositif selon la revendication 2, dans lequel la force de déviation est fournie par la résilience de l'élément indicateur.

4. Dispositif selon la revendication 3, dans lequel l'élément indicateur résilient est un ressort qui est fixé à l'élément de rupture, le ressort étant sous compression, de sorte que l'élément de rupture est sous tension.

5. Dispositif selon l'une quelconque des revendications précédentes, dans lequel l'élément de rupture est un élément tubulaire.

6. Dispositif selon la revendication 5, dans lequel l'élément tubulaire est scellé, l'intérieur de l'élément tubulaire est maintenu à une pression autre que la pression atmosphérique, et des moyens sont fournis pour surveiller cette pression afin de déterminer l'intégrité de l'élément tubulaire.

7. Dispositif selon la revendication 4, dans lequel le ressort est fixé à l'élément de rupture par une rondelle frein en étoile respective à chaque extrémité du ressort, chaque rondelle étant fixée à l'élément de rupture de manière à pouvoir se déplacer uniquement dans une direction le long de l'élément de rupture.

8. Dispositif selon la revendication 1, dans lequel l'élément de rupture est constitué d'un matériau qui change d'apparence en présence de la fuite.

9. Dispositif selon la revendication 1, dans lequel l'élément indicateur est maintenu dans la première position par une force de déviation et dans lequel une autre force, qui est assez forte pour dépasser la force de déviation est agencée pour agir sur l'élément indicateur afin de le déplacer dans la seconde position lors de la rupture de l'élément de rupture.

10. Dispositif selon la revendication 9, dans lequel l'élément de rupture est un élément tubulaire et l'élément indicateur est à l'intérieur de l'élément tubulaire et est fixé à une extrémité de l'élément de rupture, tandis que son autre extrémité se projette au-delà de l'autre extrémité de l'élément de rupture et est déviée à distance de l'autre extrémité de l'élément de rupture.

11. Dispositif selon la revendication 9, dans lequel l'élément de rupture et l'élément indicateur sont agencés pour être supportés verticalement et dans lequel l'autre force est la gravité.

12. Dispositif destiné à détecter la présence d'une fuite chimique, le dispositif comprenant un élément indicateur résilient qui est maintenu dans une première position et qui est fixé dans la première position au moyen d'un l'élément de rupture, l'élément de rupture étant constitué d'un matériau qui casse en présence de la fuite chimique, libérant ainsi l'élément indicateur de sa première position et lui permettant de se déplacer dans une seconde position afin de fournir une indication de la présence de la fuite ; dans lequel l'élément de rupture est allongé dans le sens où il est plus large dans la direction dans laquelle l'élément indicateur se déplace lors de la rupture de l'élément de rupture qu'il ne l'est dans aucune autre dimension, et dans lequel l'élément de rupture comprend un nombre de différents matériaux agencés en série et/ou en parallèle.

13. Dispositif selon la revendication 12, dans lequel l'élément de rupture est maintenu sous tension.

14. Dispositif selon la revendication 12, dans lequel l'élément indicateur est maintenu dans la première position par une force de déviation, la force de déviation agissant pour déplacer l'élément indicateur dans la seconde position lors de la rupture de l'élément de rupture.

15. Dispositif selon la revendication 14, dans lequel la force de déviation est fournie par la résilience de l'élément indicateur.

16. Dispositif selon la revendication 15, dans lequel l'élément indicateur résilient est un ressort qui est fixé à l'élément de rupture, le ressort étant sous compression, de sorte que l'élément de rupture est sous tension.

17. Dispositif selon la revendication 12, dans lequel l'élément de rupture est un élément tubulaire.

18. Dispositif selon la revendication 17, dans lequel l'élément tubulaire est scellé, l'intérieur de l'élément tubulaire est maintenu à une pression autre que la pression atmosphérique, et des moyens sont fournis pour surveiller cette pression afin de déterminer l'intégrité de l'élément tubulaire.

19. Dispositif selon la revendication 16, dans lequel le ressort est fixé à l'élément de rupture par une rondelle frein en étoile respective à chaque extrémité du ressort, chaque rondelle étant fixée à l'élément de rupture de manière à pouvoir se déplacer uniquement dans une direction le long de l'élément de rupture.

20. Dispositif selon la revendication 12, dans lequel l'élément de rupture est constitué d'un matériau qui change d'apparence en présence du contaminant.

21. Dispositif selon la revendication 12, dans lequel l'élément indicateur est maintenu dans la première position par une force de déviation et dans lequel une autre force, qui est assez forte pour dépasser la force de déviation est agencée pour agir sur l'élément indicateur afin de le déplacer dans la seconde position lors de la rupture de l'élément de rupture.

22. Dispositif selon la revendication 21, dans lequel l'élément de rupture est un élément tubulaire et l'élément indicateur est à l'intérieur de l'élément tubulaire et est fixé à une extrémité de l'élément de rupture, tandis que son autre extrémité se projette au-delà de l'autre extrémité de l'élément de rupture et est déviée à distance de l'autre extrémité de l'élément de rupture.

23. Dispositif selon la revendication 22, dans lequel l'élément de rupture et l'élément indicateur sont agencés pour être supportés verticalement et dans lequel l'autre force est la gravité.

24. Agencement destiné à détecter la présence d'une fuite chimique sur une zone prédéterminée, l'agencement comprenant une pluralité de dispositifs selon l'une quelconque des revendications précédentes agencés sur la zone.

25. Agencement selon la revendication 24, dans lequel les dispositifs sont agencés de manière sensiblement parallèle.

26. Procédé de détection d'une fuite chimique comprenant les étapes consistant à positionner un dispositif selon la revendication 1, dans un site de fuite chimique potentielle et à surveiller l'élément de rupture pour déterminer le moment où il s'est déplacé dans la seconde position indiquant la présence d'une fuite.

27. Procédé de détection d'une fuite chimique comprenant les étapes consistant à positionner un agencement selon la revendication 24, dans un site de fuite chimique potentielle et à surveiller l'élément de rupture pour déterminer le moment où il s'est déplacé dans la seconde position indiquant la présence d'une fuite.

28. Procédé de détection de fuites provenant d'une cuve dans une station service contenant une source potentielle de contaminant chimique, le procédé comprenant les étapes consistant à positionner un dispositif selon la revendication 1, dans le sol en dessous de la cuve ; et à surveiller l'élément de rupture pour déterminer le moment où il s'est déplacé dans la seconde position indiquant la présence d'une fuite.

29. Procédé de détection de fuites provenant d'une cuve dans une station service contenant une source potentielle de contaminant chimique, le procédé comprenant les étapes consistant à positionner un agencement selon la revendication 24, dans le sol en dessous de la cuve ; et à surveiller chaque élément de rupture pour déterminer le moment où il s'est déplacé dans la seconde position indiquant la présence d'une fuite.
